# EUROPEAN PATENT SPECIFICATION

(11) **EP 2 464 314 B1**
(45) Date of publication and mention of the grant of the patent: **23.12.2015**
(21) Application number: 10749820.6
(22) Date of filing: 10.08.2010
(51) Int. Cl.: A61F 2/38

(54) **KNEE PROSTHESIS**
Knieprothese
Prothèse de genou

(30) Priority: 10.08.2009 EP 09167563
(43) Date of publication of application: 20.06.2012
(73) Proprietor: Orthopaedic Innovation Centre Inc., Winnipeg MB R2K 2M9 (CA)
(72) Inventor: Wyss Urs, Winnipeg, MT R2H 0E9 (CA); Amiri Sharam, Vancouver, BC V6E 4R3 (CA); Cooke Theodore Derek Vernon, Perth, ON K7H 3A5 (CA)
(74) Representative: Dr. Graf & Partner AG
(86) International application number: PCT/EP2010/061572
(87) International publication number: WO 2011/018441

(56) References cited:
- EP-A1- 1 159 938
- WO-A1-2005/044150
- WO-A2-2007/119173
- GB-A- 2 253 147
- US-A- 6 013 103
- US-A1- 2004 186 582

## Description

The invention covers a knee prosthesis for a knee joint of a width "w" with a ball-like femoral condyle and a corresponding tibial cavity on the medial side, the ball and cavity having a centre "Mb", a radius "Rb" , a spherical surface "Sb" and defining a Cartesian coordinate system X, Y, Z attached to the Tibia with its origin "O" at the centre "Mb".

The differences in the functional outcomes of the subjects with normal knees and those who have undergone total knee replacement (TKR) have been linked to different kinematics between normal and the artificial knees (Victor and Bellemans, 2006; Andriacchi T.P and Dyrby C.O, 2005). Medial Pivot knees incorporate a congruent ball and socket configuration on the medial compartment of the joint, which replicates the anatomic configuration of the normal knee joint and allows for accommodation of the natural patterns of motion. These knees, however, in the absence of the cruciate ligaments do not have any guiding features to control the motion of the joint into a normal pattern. To have a TKR with normal kinematics, a design concept for a surface-guided TKR with converged femoral condyle has been proposed by P.S.WALKER (Bearing Surfaces for Motion Control in Total Knee Arthroplasty; Total Knee Athroplasty: A Guide to Get Better Performance edited by Bellemans J, Ries M.D., Victor J. /Pp 295- 302; Springer Berlin Heidelberg); an especially-shaped bearing surface, in particular with converged bearing spacing of the femoral condyles, was suggested as a design feature for controlling the motion of the joint. Combinations of ramp features and an anterior recess/pad have also been suggested as potential designs for producing normal kinematics (Walker et al., 2007).

It is the aim of this invention to create an alternative knee prosthesis, which comes close to the motion characteristics of a natural knee. This is achieved with the features of the independent claim 1
- in that on the lateral compartment exists a trace-line "Lt1" of contact points "Pt1" for the tibia component as a predetermined curve on a spherical surface "Sc1", which has its centre at the origin "O" and which has a radius in a range "Rc1" = 0,65w +/- 0,25w
- in that at a given flexion angle γ for each contact point "Pt1" there exists on a trace-line "Lt2" a common contact point "Pt2" for a spherical surface "Sc2" attached to the femur, which is identical to "Sc1" and has origin "O" and radius "Rc2"= Rc1, whereby at a given flexion angle γ there exists a plane "E1" through origin "O" and the common contact point Pt1/Pt2, which contains the same guiding curves "Bi", "Be" on the tibial and on the femoral part, "Bi" towards medial and "Be" towards lateral, both of which stay in a geometrically fixed relation to the common contact point Pt1/Pt2,
- and in that at least one of the guiding curves "Bi" and "Be" progressively changes its shape by change of flexion angle γ, for generating an enforced gliding and rolling movement in flexion direction or in extension direction or preferably that the guiding curves "Bi" and "Be" progressively change their shapes in opposite directions by change of flexion angle γ, for generating an enforced gliding and rolling movement in both flexion and extension directions.

The invention has the advantage that there is a clear guidance for pivoting the tibia about the medial side of the femur as a function of the flexion angle γ as long as there is joint compression applied due to muscle forces, weight, and ligament tensions to enforce contact. A very important feature of the invention is that to satisfy the kinematic degrees of freedom and the geometric constraints required for proper guiding, the geometries of the medial and lateral sides are designed in harmony with the characteristics of the expected kinematics. It can be shown that proper guiding can only take place by considering certain relationships between the characteristics of the expected kinematics and the geometries of the ball-shape medial compartment and guiding surfaces of the lateral compartment. In other words, in contrary to the invention there would be no proper kinematic control enforced if the shapes of the condyles are designed simply by incorporating variations in the geometric parameters (i.e converged bearing distance of the condyles), without taking into account the expected kinematics patterns (i.e the exact direction of rolling and sliding of the surfaces) during design of both femoral and tibial components.

The locations of contact on the guiding surfaces do not interfere with the locations of cruciate ligaments and with the location of the patellar groove on the femur. Therefore, there can be options for preserving the cruciate ligaments and for having anatomical shape for the trochlear groove on the femur, which enhances natural articulation of the patella for the full range of motion from full extension to deep flexion.

Another advantage of the invention is that it clearly defines the complete geometric relationships required to generate the complex 3D geometries of the guiding surfaces, which facilitates the production of components through numerically controlled machine tools for instance. Since geometric relationships are defined dimensionless as functions of the width of the knee joint, they can be used to generate any size of the prosthesis, when the width is known.

The dependent claims 2 to 13 show improvements of the invention. If the guiding curves "Bi" and "Be" are arcs geometries of which are defined based on mathematical functions, programming of the guiding surface becomes easier. These arcs can be parts of the circles tangent at the common contact point Pt1/Pt2 to the line "T2" in plane E1 which is drawn from the common contact point to the surface "Sb" of the ball.

Another object of the invention is to generate a three-dimensional trace-line "Lt1" according to measurements on cadaveric specimen but with a simple generator. This is achieved by defining a generator in a sagittal plane and by projecting it orthogonally from the sagittal plane to the spherical surface "Sc1" of the tibia component. The generator may be a continuous curve located between two circular boundaries with radii R1 and R2, which have a common centre "Ms" with the coordinates defined with respect to the aforementioned coordinate system (x = 0,07w ; y = - 0,794w ; z=0,5w) and the radius R1 = 0,54w + 0,08w respectively radius R2 = 0,54w - 0,08w. As the cadaver tests vary from one specimen to another, a middle range for the limiting radii is proposed with R1 = 0, 54w + 0,03w and R2 = 0,54w - 0,03w. A good approach is already one circle with a radius R= 0,54w.

Another object of the invention is to have clearly defined relative movements between the tibia and femur as rotation about a 3D axis passing through the centre of the medial ball. This is achieved in that at each flexion angle γ a tangent line T1 to the trace of tibial contact points Lt1 at the contact point Pt1 is also the tangent to the trace line Lt2 on the spherical surface "Sc2" of the femur, and in that a momentary rotational axis for the movement between the two spheres Sc1/Sc2 is therefore located on a plane E1, which is perpendicular to the common tangent T1. Knowing the exact kinematics that should be generated by the surfaces through their interactions, for each flexion angle γ the orientation of the momentary axis of rotation and also the magnitude of incremental rotation about this axis can be calculated. This information will be used to generate a trace of contact points on the femur Lt2 that matches the corresponding trace of contact on the tibia Lt1. For this purpose, starting from the upright position where the location of Pt1 and Pt2 is at the most anterior point of Lt1, the location of Pt2 with respect to the femur will be recorded and an increment of flexion angle will be imposed to the femur about the momentary rotation axis located on the plane E1 and oriented as prescribed by the input kinematics. This will move both of the contact points Pt1 and Pt2 to new matching locations on Lt1 and Lt2. The new location of the contact point on Lt2 with respect to the femur will be stored and the motion will continue until the end. Connecting the group of Pt2 points stored for various flexion angles will define the Lt2 curve.

The input kinematics in this process can be taken from cadavers or living subjects. However, the input kinematics should be processed to first meet the geometric constraints of the ball and socket configuration with minimum deviation from its original form. Second the processed kinematics should be able to generate a trace of contact points in proximity to the surface of the cartilage in the normal knee joint, so that as a further advantage minimum bone resection would be required during implantation. A thesis of the inventor Shahran Amiri (not yet public; *Title :* Conceptual Design for a Surface-Guided Total Knee Replacement with Normal Kinematics; 2009, Queen's University, Kingston, Canada) shows how optimization methods can be used to process the input kinematics and generate corresponding articular surfaces. It further shows, that the certain geometric compatibility should be maintained between the design of the medial and lateral compartment.

The suggested guiding features have the advantage of not interfering with the geometric locations of the patellofemoral articulation and also the location of the cruciates and their attachments. This offers the options for bi-cruciate or PCL-retaining designs as well as a design with a more normal patellofemoral articulation throughout the full range of motion.

Since a natural knee has a congruent configuration similar to what the medial ball-and-socket design suggests, a lateral monocompartmental prosthesis can also be developed based on the introduced concept. Another solution can be a prosthesis with two monocompartmental parts, a ball-like medial compartment and a lateral compartment which incorporates the introduced guiding features.

The invention offers a method for constructing a knee prosthesis for a knee joint of a width "w" with a ball-like femoral condyle and a corresponding tibia cavity on the medial side, the ball and the cavity having a centre "Mb", a radius "Rb", a spherical surface "Sb" and defining a Cartesian coordinate system X, Y, Z attached to the tibia with its origin "O" at the centre "Mb",
- whereby in a first step on the lateral compartment a trace-line "Lt1" of contact points "Pt1" for the tibia component is generated as a predetermined curve on a spherical surface "Sc1", which has its centre at the origin "O" and which has a radius in a range "Rc1"= 0,65w +/- 0,25w;
- whereby at a given flexion angle γ for each contact point "Pt1" there exists on a trace-line "Lt2" a common contact point "Pt2" for a spherical surface "Sc2" attached to the femur, which is identical to "Sc1" and has origin "O" and radius "Rc2"= Rc1;
- whereby at a given flexion angle γ there exists a plane "E1" through origin "O" and the common contact point Pt1/Pt2, which contains the same guiding curves "Bi", "Be" on the tibial and on the femur part, "Bi" towards medial and "Be" towards lateral, both of which stand in a geometrical fixed relation to the common contact point Pt1/Pt2;
- whereby at each increment of flexion a corresponding magnitude of increment for a pivoting angle β with respect to flexion angle γ is taken from a known relationship β as a function of γ for a further point Pt2 of the trace-line Lt2, which falls together with the new point Pt1 on the predetermined curve, which represents the trace-line Lt1;
- constructing thus the trace-line Lt2 by incremental steps and adding the guiding curves Bi ad Be for each pair of the corresponding angles γ and β in the corresponding plane E1;
- and finally generating guiding curves Bi and Be for the femoral and the tibial part over the full range of γ and β in a density, which allows machining of the lateral guiding surfaces for tibia and femur for instance by NC machine tools.

The predetermined curve may be generated by interference on the spherical surface "Sc" with a the surface of a cylinder , which stands orthogonal to a sagittal plane and which is constructed by a continuous curve "Lc" located on the sagittal plane:
- The continuous curve Lc, used to construct the cylinder here is lying on the sagittal plane and between two circular boundaries with radii R1 and R2, which have a common centre "Ms" with the coordinates X= 0,07w; Y=-0,794w; Z= 0,5w and the radius R1= 0,54w + 0,08w respectively radius R2= 0,54w - 0,08w. These dimensions may be restricted to a radius R1 taking R1= 0,54w + 0,03w and radius R2 taking R2=0,54w - 0,03w.

It is understood, that at each flexion angle γ a tangent T1 to the trace-line Lt1 at the contact point Pt1 is also the tangent for the trace-line Lt2 on the spherical surface "Sc2" of the femur and that at each flexion angle the location of a momentary rotation axis is on a plane E1, which passes through the centre Mb of the medial ball and is perpendicular to the tangent T1 of the three-dimensional trace-line Lt1 at contact point Pt1 for the tibia.

The guiding curves "Bi" and "Be" may progressively change their shapes in opposite directions by change of flexion angle γ, for generating an enforced gliding and rolling movement in both flexion and extension directions. Additionally conical surfaces can be added on the interior side of the trace-lines Lt1 and Lt2 for additional support, which have their centres at the centre Mb of the medial ball and which have the trace-lines Lt1, Lt2 as generator for the cones.

The guiding curves "Be" and "Bi" may be arcs, which start from common contact points Pt1/Pt2. In order to come close to the location of natural guiding surfaces, the guiding curves "Be" and "Bi" at the contact points Pt1/Pt2 may be tangent to a line "T2" on the plane E1, which is drawn from the common contact point Pt1/Pt2 to the surface "Sb" on the ball, whereby the plane E1 is orthogonal to a tangent "T1" of the trace-line Lt1 at the common contact point Pt1/Pt2.

The guiding curves "Be" and "Bi" may be circular arcs with radii "Re" and "Ri" and the curves for the tibial component may be less congruent to the corresponding guiding curves of the femoral component in the middle range of flexion angle γ, than for the end positions full extension and full flexion.

Preferred embodiments of the invention are described below with reference to the drawings, wherein like numerals are used to refer to the same or similar elements:
- Fig. 1:: shows schematically a posterior view of a knee joint prosthesis with a coordinate System X; Y; Z;
- Fig. 2a:: shows schematically a vertical cut of a tibia component along the YZ-plane;
- Fig. 2b:: shows schematically a view from lateral of Figure 2a with a cut along a trace-line Lt1 on a spherical surface Sc1, as well as a generating curve Lc in a sagittal plane 4;
- Fig. 2c:: shows schematically a top view of the tibia component of Figure 2b and 2c with a trace-line Lt1;
- Fig. 3:: shows graphically for the lateral sagittal plane the angular location of contact points Pt1 with the angle α as a function of the flexion angle γ and also some values for the angular relationship in a table;
- Fig. 4:: shows a schematic view of the tibia component 2 with plane E1, which passes through a the centre O, and the contact point Pt1, perpendicular to the tangent of the trace-line Lt1 at the point Pt1;
- Fig. 5:: shows graphically the pivot angle P of the femur with reference to the tibia as a function of the flexion angle γ, and also some values for the angular relationship in a table;
- Fig. 6:: shows schematically from posterior a plane E1 with the geometrical relationship between the lateral guiding curves Be and Bi and the medial spherical surface Sb;
- Fig. 7:: shows a table with the dimensions of the radii for the lateral guiding curves and their corresponding flexion angles γ;
- Fig. 8a:: shows schematically a view from posterior for an extended position;
- Fig. 8b:: shows schematically the changes in the shape of the guiding curves for different flexion angles γ;
- Fig. 9:: shows schematically for femur and tibia a graph with the interior radius Ri as a fraction of the width w depending on the flexion angle γ;
- Fig. 10:: shows schematically for femur and tibia a graph with the exterior radius Re as a fraction of the width w depending on the flexion angle γ;
- Fig. 11:: shows schematically a femur prosthesis part from distal with an additional conical surface;
- Fig. 12:: shows schematically from a posterior angle a knee prosthesis with the additional conical surfaces;
- Fig. 13:: shows schematically a cut through a tibial part along a plane E1 at full extension with the bearing surfaces; and
- Fig. 14:: shows schematically a cut through the tibial part of Figure 13 along a plane E1 for a large flexion angle with the bearing surfaces.

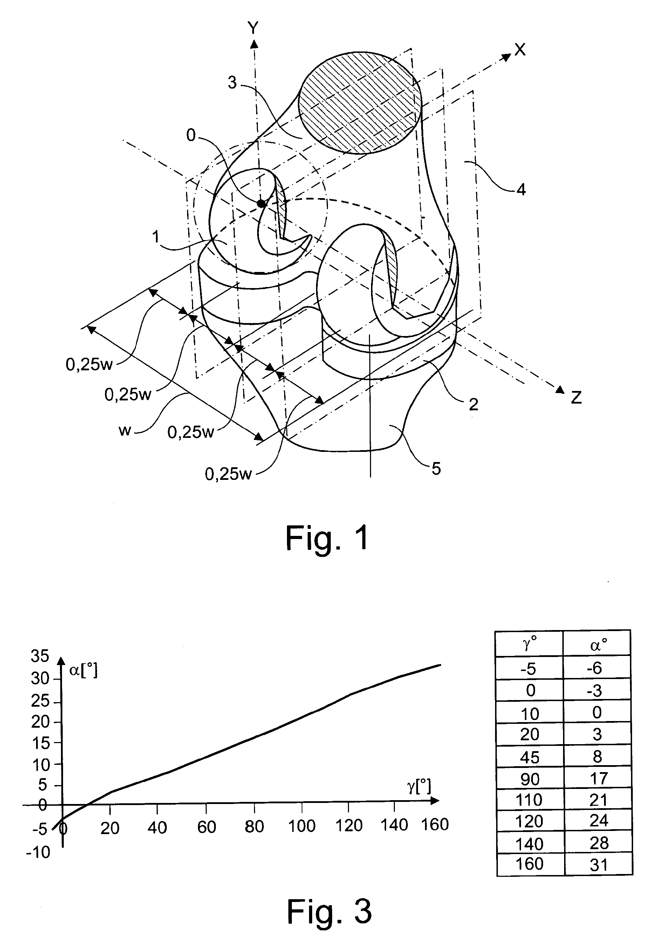

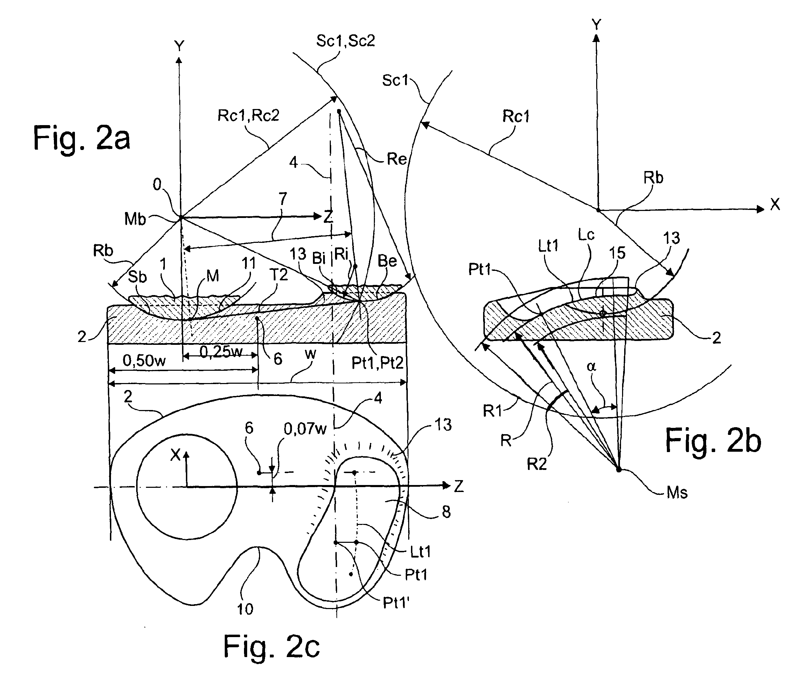

In Figure 1 femur 3 and tibia bone 5 are shown with their prosthetic parts in extension position. The parameter "w" defines the width of the component 2 in the mediolateral direction. The medial and lateral sagittal planes 4 are defined parallel to the sagittal plane of the tibia 5 and passing through the medial and lateral centre of the tibia plateau 2 at a distance of 0,25w from the midpoint 6 (see Figure 2a) of the width w. A coordinate system X, Y, Z has its centre O on the medial sagittal plane. The X,Y, and Z axes in this coordinates system point to the anterior, proximal, and lateral directions respectively.

In Figures 2a, 2b and 2c the position and the shape of a lateral cavity 8 are illustrated. Starting from the midpoint 6 of the line that connects the medial and the lateral centre of the tibia component, a reference coordinate system is defined having its origin "O" by 0,25w in medial direction, by 0,32w in proximal direction and by 0.07w in posterior direction. From origin "O" the X-axis points towards anterior, the Y-axis points towards proximal and the Z-axis points towards lateral. The centre "Mb" of the ball-like medial condyle 1 is at the origin "O" and the radius of the medial ball is Rb=0,32w.

The shapes of the lateral condyles are generated by first defining the trace-lines Lt1, Lt2 of contact points Pt1, Pt2 on the lateral compartment. Two identical spherical surfaces are defined, one "Sc1" attached to the tibia and one "Sc2" attached to the femur. These spheres are defined concentric with the medial ball when the joint is at full extension and with their radii Rc1 and Rc2 equal to 0,65w. The trace-lines Lt1 and Lt2 of contact points are both located on the corresponding spherical surface Sc1 and Sc2. As can be seen in Figure 2a at the common contact points Pt1/Pt2 on the lateral side, the shapes of the guiding curves Be, Bi for the tibial and femoral condyles are identical. During the motion of the joint, the two reference spheres Sc1 and Sc2 always remain concentric, causing the spheres to slide on top of each other while tangency between the trace-lines Lt1, Lt2 is maintained.

The three dimensional trace-line Lt1 of contact points Pt1 is generated by projecting a two dimensional curve in the lateral sagittal plane 4 on the spherical surface Sc1 of the tibia component in the mediolateral direction. In this example (Fig. 2b) the curve is a circular arc in the lateral sagittal plane 4, having a radius R=0,54w and its centre Ms is located at x=0,07w; y=- 0,794w; z=0,5w defined with respect to the above-mentioned coordinate system. This circular arc lies between two boundaries defined by two arcs with the same centre and with radius of R1=0,54w + 0.08w and R2=0,54w - 0,08w as shown in Figure 2b. The location of a projected contact point Pt1 on the arc Lc is driven from the point Pt1' (Fig. 2c) on the medial sagittal plane location of which is defined by angle α defined between the reference line that passes through the point Pt1' and the centre of the arc and a proximal-distal reference line on the lateral sagittal plane (Fig. 2b). So for each flexion angle the contact point on the lateral sagittal plane Pt1'and the corresponding Pt1 is defined by the arc and a corresponding angle α. Therefore the 3D trace of contact points on the tibia Lt1 is produced by projecting the arc Lc on the Sphere Sc1. It is understood that the arc Lc on the sagittal plane is part of a cylindrical surface, which stands orthogonal to the sagittal plane and which interferes with the spherical surface Sc1.

In Figure 3, for flexion angles γ ranging from -5° to 160°, the values for angle α are shown in a graph and in a table. With the flexion angle γ increasing the lateral contact point Pt1 continuously moves in the posterior direction along the trace-line Lt1 (Fig.2b, Fig.2c). When the motion is reversed the contact point Pt1 will sweep the same exact path in the opposite direction.

The matching trace of contact points on the femur is created by keeping the tibia fixed, and incrementally moving the femur with respect to the tibia starting from -5° of flexion and finishing at 160°, following the desired kinematics; in each increment the point Pt1 on the trace-line Lt1 of contact points of the tibia which is associated with the current flexion angle is added as a contact point Pt2 to the femoral sphere. The motion continues until 160°, and at the end all the Pt2 points added to the femur form the trace-line Lt2 of contact points on the femur. Because of the identical geometries of the tibia and femoral spheres, the trace of contact points Lt2 on the femur is exactly placed over the femoral sphere Sc2.

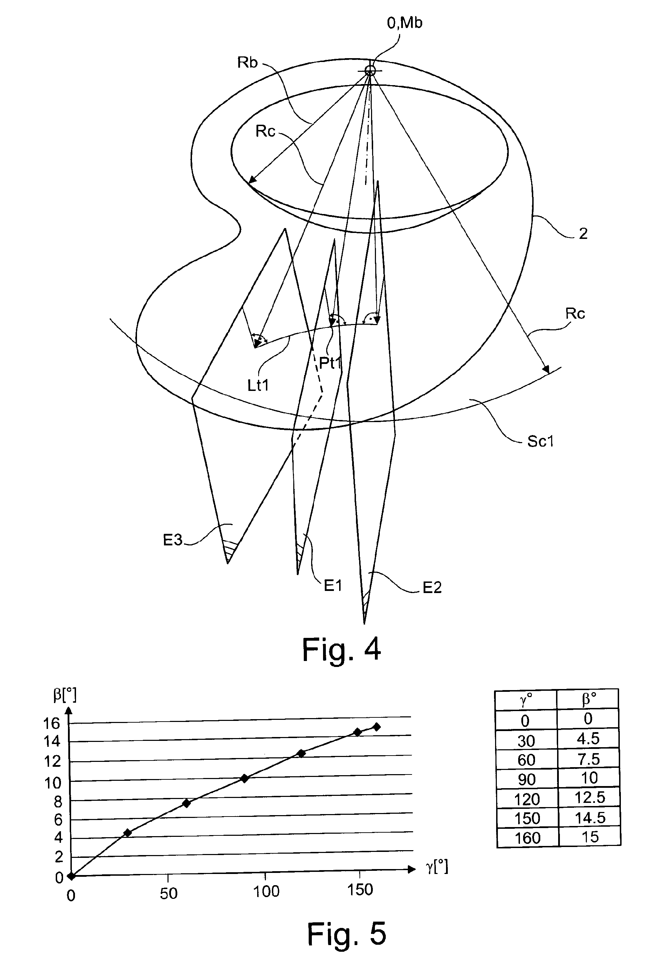

In conformity with the cited literature the flexion axis is on a plane E1 parallel to the XZ plane of the tibia and the pivoting axis is defined perpendicular to the flexion axis on reference plane E1. So cadaver tests and results, as shown in Fig.3 and Fig.5, are adapted to be used as inputs for mathematical modelling of implant surfaces.

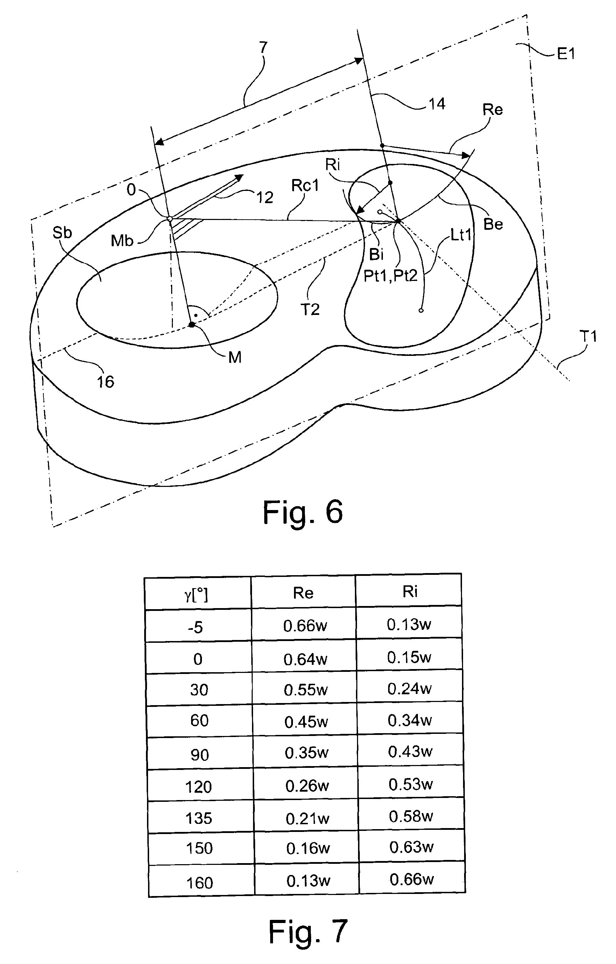

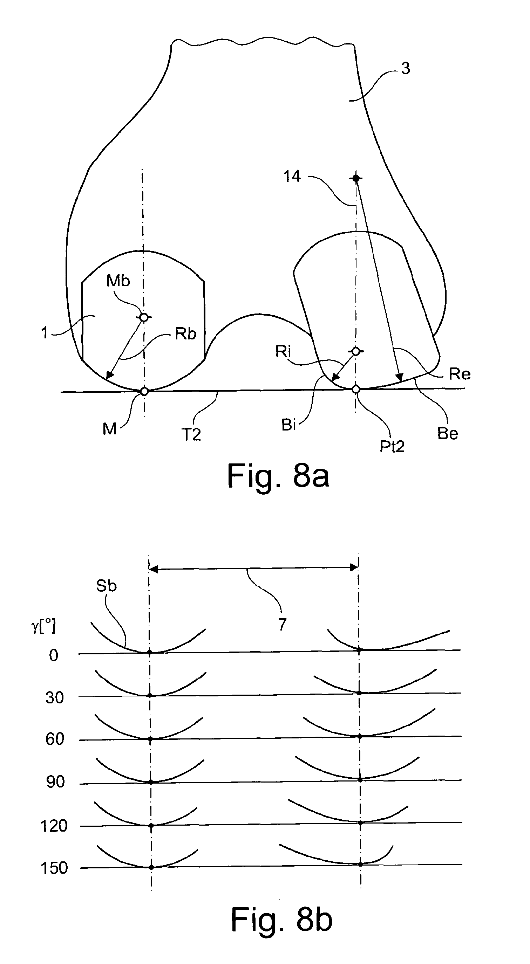

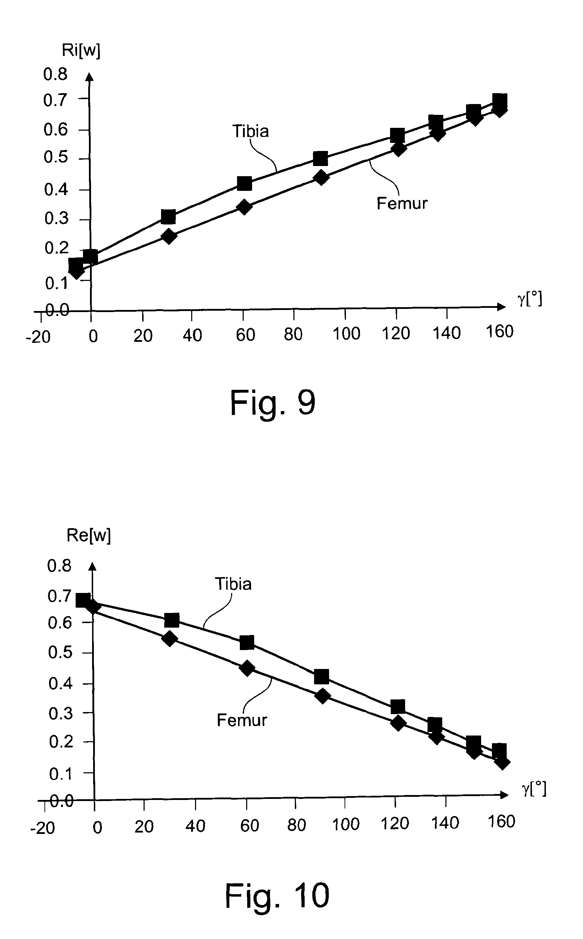

Looking at Figures 4 and 6: At each moment of the motion, the locations of contact points on the tibia Pt1 and femoral sphere Pt2, which are associated with the current flexion angle γ, will be identical (also written as Pt1/Pt2).
- The motion from -5° of flexion to 160° flexion for example is broken down into 25 increments (increment of flexion is calculated as 165/25=6,6°).
- From one increment to another, the femur rotates about the centre of the medial ball.
- At each flexion angle the location of the momentary rotation axis 12 is on the plane E1, which passes through the centre Mb of the medial ball 1 and is perpendicular to the common tangent T1 to the three-dimensional trace-line Lt1 at contact point Pt1 for the tibia and the trace-line Lt2 at contact point Pt2 for the femur. The planes E2 anE3 show the end joint positions, full extension and full flexion (Figure 4).
   1- Rotation of the femur with respect to the tibia in each increment is assumed to have two components namely flexion and pivoting.
   2- The orientation of the momentary flexion axis is assumed on the plane E1, passing through the centre of the medial ball and parallel to the XZ plane.
   3- The orientation of the pivot is considered on the plane E1, passing through the centre of the medial ball and parallel to the XY plane.
   4- For each increment of motion , the location of the contact point Pt1 is defined as explained before. The size of the contact arc can be scaled to accommodate variations in the anteroposterior to mediolateral width of knee joints because of gender and ethnic differences.
   5- The magnitude of incremental flexion is 6.6° as described before. The corresponding magnitude of increment for each pivoting angle b with respect to the tibia is extracted from graph and table of Figure 5. This graph shows how the pivoting of the tibia takes place as the flexion angles γ increases/decreases. [It is understood that the graphs of Figure 3 and of Figure 5 can be approached by mathematical continuous functions, for instance polynomial equations within the shown limits. It is also understood, that these graphs and functions can be slightly varied to obtain different results for the trace-line Lt1 on the tibia and the trace-line Lt2 one the spherical surface Sc2 of the femur]
   6- At each increment of motion, the guiding curves Be and Bi of the tibia and femur on the lateral side are defined on the plane E1 (see Figure 6). The guiding curves Be and Bi of the tibia and of the femur pass through the momentary common contact points Pt1/Pt2. As mentioned before, plane E1 passes through the centre Mb of the medial ball and stands orthogonal to the trace-line Lt1 at the contact point Pt1 of the tibia as well as orthogonal to the trace-line Lt2 at the same contact point Pt2 of the femur. Two reference lines are constructed on plane E1. The tangential reference line T2 is considered on plane E1 as the line that passes through the point of contact Pt1/Pt2 and is tangent at point M to the medial spherical surface Sb. An orthogonal reference line 14 on plane E1 is considered as a line passing through the contact point Pt1/Pt2 and perpendicular to the tangential reference line T2.
   7- Due to the described geometric relationships, at each moment of motion there exists a triangle (Pt1/Pt2-O-M) on plane E1 with the shape that always remains unchanged during the motion. Because of this, the bearing spacing 7 which is the distance between the medial contact point M and the lateral contact pointPt1/Pt2 will always remain constant during the motion (see also Fig. 2a).
   8- In Figure 6 the plane E1 cuts through the tibial component and the intersecting line at the top surface is shown by a phantom line 16. The shape of the guiding curves Be and Bi on the lateral side for the femur and for the tibia component is a combination of two circular arcs on both sides of the contact point Pt1/Pt2. These arcs are called interior and exterior arcs, labelled according to their radii as Ri and Re, respectively. These arcs are tangent to the line T2 at their starting point Pt1/Pt2 and centres of the arcs lie on the orthogonal reference line 14.
   9- The radii Ri and Re of these guiding curves "Bi" and "Be" progressively change their shapes in opposite directions with changing flexion angle γ. This generates an enforced gliding and rolling movement in both directions, as the femoral trace-line Lt2 is much longer than the tibial trace-line Lt1. Figure 8a shows a posterior view with the definition of the curves Bi and Be. In Figure 8b the guiding curves Bi and Be for different flexion angles γ are drawn on top of each other to make the changes visible. As the spacing 7 between contact point Pt1/Pt2 and point M remains constant, the deepest medial and lateral points are on top of each other, if the reference line T2 is drawn horizontally for the comparison.
   10- The dimensions of the radii of the interior and of the exterior guiding curves on the lateral side are defined as functions of the flexion angle γ as described in the table of Figure 7. The values are shown dimensionless as decimal fractures of the width w.
   11-As shown in the table, the direction of growth in radius for the interior guiding curve Bi is opposite to that of the exterior guiding curve Be.
   12- The guiding curves on the lateral side for tibia and femur on the plane E1 at the common contact points Pt1/Pt2 are constructed based on the same principle. Once a satisfactory shape for trace-lines Lt1 and Lt2 as well as for the radii Re and Ri have been found - the guiding curves Be and Bi also have to match the thickness of the tibia component - the increments in the flexion could be chosen much smaller to have a sufficient description of the surface for manufacturing. Sometimes it might be helpful to have a rim 13 on the lateral side (see Figure 2) for enlarging the guiding surfaces Be and Bi.
   13- As illustrated in the graphs of Figure 9 and 10, the radii of the guiding curves on the tibia are considered larger compared to the dimensions of their corresponding femoral counter parts; this allows for clearance and controlled laxity at different flexion angles.
   14- The clearance between the tibia and femoral contact curves allows laxities. The magnitudes of these laxities are smaller for the full extension and full flexion positions of the joint. For the mid-range of motion these laxities are larger, due to larger clearances incorporated into the corresponding parts of the contact surfaces.

The trace-lines Lt1 and Lt2 are very important as they are the basis for the definition of the guiding surfaces. Nevertheless they must not be part of the guiding surfaces and could be left out. They can be virtuel for the definition of the guiding surfaces and for the relative motion between femur and tibia.

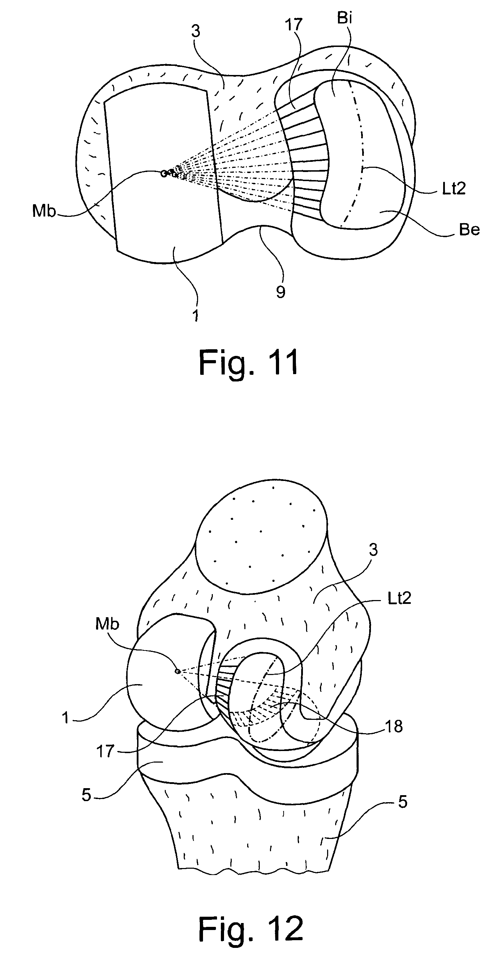

With the trace-lines Lt1 and Lt2 there can also be defined underlying conical surfaces 17, 18 that control the rolling-gliding of the surfaces: Two cones with their centres at the centre Mb of the medial ball 1 roll and glide over the top of each other. The first cone for the tibia has the trace-line Lt1 as generator for the conical surface 18; the second cone for the femur has the trace-line Lt2 as generator for the conical surface 17. For these guiding surfaces on the two cones there is rolling and gliding possible but not enforced particularly by the cones. To some extent the cones can serve as auxiliary supporting surfaces at the lateral interior side of the trace-lines Lt1 and Lt2 in combination with the above described enforced gliding and rolling system. Figure 11 is a view from approximately distal, which shows a conical surface 17 at the lateral femoral condyle. A band of this conical surface 17 could provide additional articular surface and support to its matching tibial counterpart 18, whereas the relation between gliding and rolling is controlled by the guiding curves Bi and Be. In Figure 12 the conical surface 18 is shown on the tibia component 2 as if the lateral femur part would be transparent.

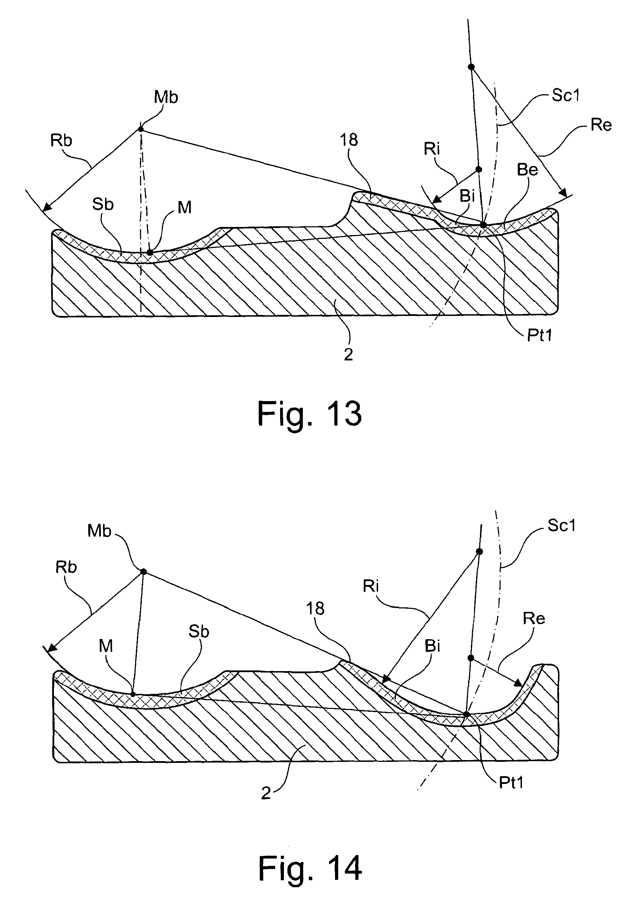

The example of the Figures 13 and 14 shows a large conical bearing surface 18 in extension and a small conical bearing surface 18 at a large flexion angle γ. This arrangement allows for sufficient bearing surface at all flexion angles.

Depending on the situation when the ACL or PCL or both are present these ligaments would be working as secondary mechanisms to guide the motion. This can produce two competing mechanisms that try to override each other in guiding the motion. To solve such a situation the clearances between the tibial and femoral guiding features can be enlarged by machining the tibial parts with slightly larger guiding curves for the medial and lateral aspects. Having different tibial parts a surgon can choose which one would suit best a patient depending on the condition of the cruciates of the particular patient.

Though a basic lateral guiding surface of the tibia can be defined mathematically, the first guiding surface, which drives from extension to flexion and the second guiding surface, which drives from flexion to extension may not be engaged simultaneously at the same flexion angle. Such guiding surfaces would form an envelope of laxities around a central path, whilst still allowing for steering effects of the contact surfaces. The range of laxities can be set correspondingly for different types of prostheses including ACL or PCL deficient knees.

Practically there are several possibilities to create a wanted laxity at the tibia side. Examples:
- with reference to Fig. 9 and Fig. 10 the radii Re and Ri could be made larger. The envelope of laxity shifts from anterior to posterior position as the flexion angle increases. The width of an envelope of laxity changes by flexion imposing more laxity for a mid-range of motion and less laxity (more stability) at small and at large flexion angles.
- with reference to the shown example in Fig. 6 the guiding curves Be and Bi could be slightly shifted in the plane E1 for a middle range in the direction of the tangent T2; the exterior guiding curve Be to spheres with a larger radius Rc and interior guiding curve Bi to spheres with a smaller radius Rc. If a third linear segment is added between the two guiding curves, then with an added laxity the joint might be riding on the third segment without engaging the two guiding curves.

If the material has enough elasticity, then the trace-line Lt1 or the third segment can deflect allowing for the side curves to partially engage and can produce traction. If the material is not elastic enough, then there could be a pinching load between the articular surfaces, that can cause surface damage to the tibia component in a long run. Elastic materials could be as an example PU, elastomeric PU, PCU, PE or UHMW Polyethylene.

**Reference signs**

| | | | |
|---|---|---|---|
| Be | guiding curve (exterior) | 1 | ball (condyle) |
| Bi | guiding curve (interior) | 2 | tibia component |
| E1 | orthogonal plane to trace-line | 3 | femur |
| E2 | orthogonal plane in extension | 4 | sagittal plane |
| E3 | orthogonal plane in full flexion | 5 | tibia |
| Lt1 | trace-line tibia | 6 | midpoint |
| Lt2 | trace-line femur | 7 | spacing |
| M | tangent point | 8 | lateral cavity |
| Mb | centre of ball | 9 | groove |
| Ms | centre | 10 | cut out |
| O | origin | 11 | cavity |
| Pt1 | contact point tibia | 12 | momentary rotational axis |
| Pt2 | contact point femur | 13 | rim |
| R | radius in sagittal plane | 14 | orthogonal reference line |
| R1 | radius in sagittal plane | 15 | curve |
| R2 | radius in sagittal plane | 16 | phantom line |
| Rb | radius of medial ball | 17 | conical surface femur |
| Rc1 | radius of tibial sphere | 18 | conical surface tibia |
| Rc2 | radius of femoral sphere | | |
| Sb | spherical surface of ball | | |
| Sc1 | spherical surface (trace-line) | | |
| Sc2 | spherical surface (trace-line) | | |
| T2 | tangent reference line | | |
| X | axis | | |
| Y | axis | | |
| Z | axis | | |
| w | width of joint | | |
| | | | |
| α | angle in the lateral sagittal plane | | |
| β | pivoting angle | | |
| γ | flexion angle | | |

## Claims

1. Knee prosthesis for a knee joint of a width "w" with a ball-like femoral condyle (1) and a corresponding tibia cavity (11) on the medial side, the ball (1) and cavity (11) having a centre "Mb", a radius "Rb" , a spherical surface "Sb" and defining a Cartesian coordinate system X, Y, Z attached to the tibia its origin "O" at the centre "Mb", and with a lateral compartmet (8), **characterized**
- **in that** on the lateral compartment exists a trace-line "Lt1" of contact points "Pt1" for the tibia component (2) as a predetermined curve (15) on a spherical surface "Sc1", which has its centre at the origin "O" and which has a radius in a range "Rc1" = 0,65w +/- 0,25w,
- **in that** at a given flexion angle γ for each contact point "Pt1" there exists on a trace-line "Lt2" a common contact point "Pt2" for a spherical surface "Sc2" attached to the femur, which is identical to "Sc1" and has origin "O" and radius "Rc2"= Rc1, whereby at a given flexion angle γ there exists a plane "E1" through origin "O" and the common contact point Pt1/Pt2, which contains the same guiding curves "Bi", "Be" on the tibial and on the femoral part, "Bi" towards medial and "Be" towards lateral, both of which stay in a geometrically fixed relation to the common contact point Pt1/Pt2,
- and **in that** at least one of the guiding curves "Bi" and "Be" progressively changes its shape by change of flexion angle γ, for generating an enforced gliding and rolling movement in flexion direction or in extension direction.

2. Knee prosthesis according to claim 1, **characterized in that** the guiding curves "Bi" and "Be" progressively change their shapes in opposite directions by change of flexion angle γ, for generating an enforced gliding and rolling movement in both flexion and extension directions.

3. Knee prosthesis according to claim 1 or 2, **characterized in that** the guiding curves "Bi" and "Be" are arcs , which start from common contact points Pt1/Pt2.

4. Knee prosthesis according one of the claims 1 or 3, **characterized in that** the plane E1 is orthogonal to a tangent "T1" of the trace-line Lt1 at the common contact point Pt1/Pt2 and **in that** the guiding curves "Bi" and "Be" at the common contact point Pt1/Pt2 are tangent to a line "T2" on plane E1, which is drawn from the common contact point to the surface "Sb" of the ball (1).

5. Knee prosthesis according to claim 1 to 4, **characterized in that** at each common contact point Pt1/Pt2 the curves "Bi" and "Be" are circular arcs with radii Ri, Re .

6. Knee prosthesis according to claim 1 to 5, **characterized in that** the predetermined curve (15) is generated by the interference of the spherical surface "Sc1" with a surface of a cylinder (5), which stands orthogonal to a sagittal plane (4) and which is constructed by a continuous curve (Lc) located on **the** sagittal plane (4).

7. Knee prosthesis according claim 6, **characterized in that** the continuous curve used to construct the cylinder is lying on a sagittal plane and between two circular boundaries with radii R1 and R2, which have a common centre "Ms" with the coordinates x = 0,07w ; y = - 0,794w; z=0,5w and the radius R1 = 0,54w + 0,08w respectively radius R2 = 0,54w - 0,08w.

8. Knee prosthesis according claim 7, **characterized in that** the radius R1 takes R1 = 0,54w + 0.03w and the radius R2 takes R2 = 0,54w - 0,03w.

9. Knee prosthesis according to claim 1 to 8, **characterized in that** at each flexion angle γ a tangent T1 to the trace-line Lt1 at the contact point Pt1 is also the tangent for the trace line Lt2 on the spherical surface "Sc2" of the femur and **in that** at each flexion angle the location of the momentary rotation axis (12) is on a plane E1, which passes through the centre Mb of the medial ball 1 and is perpendicular to the tangent T1 of the three dimensional trace-line Lt1 at contact points Pt1 for the tibia.

10. Knee prosthesis according to one of the claims 1 to 9, built as a total knee prosthesis with a femoral component, which has a groove (9) similar to an anatomic patellar groove, and with a tibia component (2) with an appropriate shape of a posterior cut out (10) for maintaining one or both of the cruciate ligaments.

11. Knee prosthesis according to one of the claims 1 to 9, built as lateral monocompartmental prosthesis adapted to maintain a natural patella and patella groove in combination with the congruent articulation between the natural medial femoral condyle and the convex geometry of the tibial and meniscal geometry.

12. Knee prosthesis according claim 1 to 11, **characterized in that** the guiding surfaces "Bi" and "Be" for the tibial and for the femoral components are less congruent for the middle range of the flexion angle γ, than for the end positions full extension and full flexion.

13. Knee prosthesis according claim 1 to 12, **characterized in that** conical surfaces 17, 18 are added on the interior side of the trace-lines Lt1 and Lt2 for additional support, which have their centres at the centre Mb of the medial ball 1 and which have the trace-lines Lt1, Lt2 as generator for the cones.

## Patentansprüche

1. Knieprothese für ein Kniegelenk mit einer Breite "w" mit einem kugelförmigen Femurkondylus (1) und einem entsprechenden Tibia-Hohlraum (11) auf der medialen Seite, wobei die Kugel (1) und der Hohlraum (11) eine Mitte "Mb", einen Radius "Rb" und eine Kugeloberfläche "Sb" aufweisen und ein kartesisches Koordinatensystem X, Y, Z definieren, befestigt an der Tibia, an ihrem Ursprung "O" in der Mitte "Mb", und mit einem seitlichen Kompartiment (8), **dadurch gekennzeichnet,**
- **dass** auf dem seitlichen Kompartiment eine Verfolgungslinie "Lt1" von Kontaktpunkten "Pt1" für die Tibiakomponente (2) als eine vorbestimmte Kurve (15) auf einer Kugeloberfläche "Sc1" existiert, deren Mitte am Ursprung "O" liegt und die einen Radius in einem Bereich "Rc1" = 0,65 w +/- 0,25 w aufweist,
- **dass** bei einem gegebenen Beugewinkel γ für jeden Kontaktpunkt "Pt1" auf einer Verfolgungslinie "Lt2" ein gemeinsamer Kontaktpunkt "Pt2" für eine Kugeloberfläche "Sc2", befestigt am Femur, existiert, die mit "Sc1" identisch ist und einen Ursprung "O" und einen Radius "Rc2" = Rc1 aufweist, wobei bei einem gegebenen Beugewinkel γ eine Ebene "E1" durch den Ursprung "O" und den gemeinsamen Kontaktpunkt Pt1/Pt2 existiert, die dieselben Führungskurven "Bi", "Be" auf dem Tibiateil und dem Femurteil enthält, "Bi" nach medial und "Be" nach lateral, wobei beide in einer geometrisch fixen Beziehung zu dem gemeinsamen Kontaktpunkt Pt1/Pt2 bleiben,
- und **dass** zumindest eine der Führungskurven "Bi" und "Be" durch Veränderung des Beugewinkels γ progressiv ihre Form verändert, zur Erzeugung einer erzwungenen Gleit- und Rollbewegung in Beugerichtung oder in Streckrichtung.

2. Knieprothese nach Anspruch 1, **dadurch gekennzeichnet, dass** die Führungskurven "Bi" und "Be" durch Veränderung des Beugewinkels γ progressiv ihre Form in entgegengesetzte Richtungen verändern, zur Erzeugung einer erzwungenen Gleit- und Rollbewegung in Beugerichtung und in Streckrichtung.

3. Knieprothese nach Anspruch 1 oder 2, **dadurch gekennzeichnet, dass** die Führungskurven "Bi" und "Be" Bögen sind, die von gemeinsamen Kontaktpunkten Pt1/Pt2 beginnen.

4. Knieprothese nach Anspruch 1 oder 3, **dadurch gekennzeichnet, dass** die Ebene E1 orthogonal zu einer Tangente "T1" der Verfolgungslinie Lt1 an dem gemeinsamen Kontaktpunkt Pt1/Pt2 ist, und dass die Führungskurven "Bi" und "Be" an dem gemeinsamen Kontaktpunkt Pt1/Pt2 eine Linie "T2" auf der Ebene E1 tangieren, die von dem gemeinsamen Kontaktpunkt zu der Oberfläche "Sb" der Kugel (1) gezeichnet ist.

5. Knieprothese nach Anspruch 1 bis 4, **dadurch gekennzeichnet, dass** an jedem gemeinsamen Kontaktpunkt Pt1/Pt2 die Kurven "Bi" und "Be" Kreisbögen mit Radien Ri, Re sind.

6. Knieprothese nach Anspruch 1 bis 5, **dadurch gekennzeichnet, dass** die vorbestimmte Kurve (15) durch Zusammentreffen der Kugeloberfläche "Sc1" mit einer Oberfläche eines Zylinders (5) erzeugt wird, die orthogonal zu einer Sagittalebene (4) steht und die durch eine kontinuierliche Kurve (Lc) auf der Sagittalebene (4) konstruiert wird.

7. Knieprothese nach Anspruch 6, **dadurch gekennzeichnet, dass** die zur Konstruktion des Zylinders verwendete kontinuierliche Kurve auf einer Sagittalebene und zwischen zwei kreisförmigen Grenzen mit Radien R1 und R2 liegt, die eine gemeinsame Mitte "Ms" mit den Koordinaten x = 0,07 w; y = -0,794 w; z= 0,5 w und dem Radius R1 = 0,54 w + 0,08 w bzw. dem Radius R2 = 0,54 w - 0,08 w aufweisen.

8. Knieprothese nach Anspruch 7, **dadurch gekennzeichnet, dass** der Radius R1 den Wert R1 = 0,54 w + 0,03 w und der Radius R2 den Wert R2 = 0,54 w - 0,03 w aufweist.

9. Knieprothese nach Anspruch 1 bis 8, **dadurch gekennzeichnet, dass** bei jedem Beugewinkel γ eine Tangente T1 zu der Verfolgungslinie Lt1 an dem Kontaktpunkt Pt1 auch die Tangente für die Verfolgungslinie Lt2 auf der Kugeloberfläche "Sc2" des Femur ist, und dass bei jedem Beugewinkel der Ort der momentanen Drehachse (12) auf einer Ebene E1 liegt, die durch die Mitte Mb der medialen Kugel 1 verläuft und senkrecht zu der Tangente T1 der dreidimensionalen Verfolgungslinie Lt1 an Kontaktpunkten Pt1 für die Tibia ist.

10. Knieprothese nach einem der Ansprüche 1 bis 9, konstruiert als Knietotalprothese mit einer Femurkomponente, die eine Einkerbung (9) ähnlich einer anatomischen Patella-Rinne aufweist, und mit einer Tibiakomponente (2) mit einer entsprechenden Form eines posterioren Ausschnitts (10) zur Beibehaltung eines der oder beider Kreuzbänder.

11. Knieprothese nach einem der Ansprüche 1 bis 9, konstruiert als laterale unikompartimentelle Prothese, ausgelegt zur Aufrechterhaltung einer natürlichen Patella und Patella-Rinne in Kombination mit der kongruenten Artikulation zwischen dem natürlichen medialen Femurkondylus und der konvexen Geometrie der Tibia- und Meniskus-Geometrie.

12. Knieprothese nach Anspruch 1 bis 11, **dadurch gekennzeichnet, dass** die Führungsflächen "Bi" und "Be" für die Tibia- und für die Femurkomponenten weniger kongruent für den mittleren Bereich des Beugewinkels γ sind als für die Endstellungen in voller Streckung und voller Beugung.

13. Knieprothese nach Anspruch 1 bis 12, **dadurch gekennzeichnet, dass** konische Flächen 17, 18 auf der Innenseite der Verfolgungslinien Lt1 und Lt2 für zusätzliche Unterstützung hinzugefügt sind, deren Mitten an der Mitte Mb der medialen Kugel 1 liegen und die die Verfolgungslinie Lt1, Lt2 als Generator für die Kegel aufweisen.

## Revendications

1. Prothèse de genou pour une articulation du genou d'une largeur « w » présentant un condyle (1) fémoral de type sphérique et une cavité (11) pour tibia correspondante du côté médial, la sphère (1) et la cavité (11) ayant un centre « Mb », un rayon « Rb », une surface « Sb » sphérique et délimitant un système de coordonnées cartésiennes X, Y, Z fixé au tibia à son origine « O » étant au niveau du centre « Mb », et présentant un compartiment (8) latéral, **caractérisée**
- **en ce que**, sur le compartiment latéral existe une ligne de trace « Lt1 » de points « Pt1 » de contact du composant (2) du tibia sous forme de courbe (15) prédéterminée sur une surface « Sc1 » sphérique, qui a son centre au niveau de l'origine « O » et qui a un rayon de l'ordre de « Rc1 » = 0,65 w +/-0,25w,
- **en ce que**, à un angle γ de flexion donné pour chaque point « Pt1 » de contact il existe sur une ligne de trac « Lt2 » un point « Pt2 » de contact commun pour une surface « Sc2 » sphérique fixée au fémur, qui est identique à « Sc1 » et a une origine « O » et un rayon « Rc2 » = Rc1, selon lequel à un angle γ de flexion donné il existe un plan « E1 » passant par l'origine « O » et le point Pt1/Pt2 de contact commun, qui contient les mêmes courbes « Bi », « Be » de guidage sur la partie tibiale et la partie fémorale, « Bi » vers la partie médiale et « Be » vers la partie latérale, les deux restant en relation géométrique fixe par rapport au point Pt1/Pt2 de contact commun,
- et **en ce que**, au moins une des courbes « Bi » et « Be » de guidage change progressivement sa forme en changeant d'angle γ de flexion, pour générer un mouvement forcé de glissement et de roulement dans la direction de la flexion ou dans la direction de l'extension.

2. Prothèse de genou selon la revendication 1, **caractérisée en ce que** les courbes « Bi » et « Be » de guidage changent progressivement leur forme dans des directions opposées en changeant l'angle γ de flexion, pour générer un mouvement forcé de glissement et de roulement à la fois dans les directions de la flexion et de l'extension.

3. Prothèse de genou selon la revendication 1 ou 2, **caractérisée en ce que** les courbes « Bi » et « Be » de guidage sont des arcs, qui partent des points Pt1/Pt2 de contact commun.

4. Prothèse de genou selon l'une quelconque des revendications 1 ou 3, **caractérisée en ce que** le plan E1 est orthogonal à une tangente « T1 » de la ligne de trace « Lt1 » au point Pt1/Pt2 de contact commun et **en ce que** les courbes « Bi » et « Be » de guidage au point Pt1/Pt2 de contact commun sont tangentes à une ligne « T2 » sur le plan E1, qui est tirée du point de contact commun à la surface « Sb » de la sphère (1).

5. Prothèse de genou selon la revendication 1 à 4, **caractérisée en ce que**, à chaque point Pt1/Pt2 de contact commun les courbes « Bi » et « Be » sont des arcs circulaires avec des rayons Ri, Re.

6. Prothèse de genou selon la revendication 1 à 5 **caractérisée en ce que** la courbe (15) prédéterminée est générée par l'interférence de la surface « Sc1 » sphérique avec une surface d'un cylindre (5), qui repose orthogonalement à un plan (4) sagittal et qui est constituée d'une courbe (Lc) continue située dans le plan (4) sagittal.

7. Prothèse de genou selon la revendication 6, **caractérisée en ce que** la courbe continue utilisée pour constituer le cylindre repose dans un plan sagittal et entre deux limites circulaires avec les rayons R1 et R2, qui ont un centre « Ms » commun avec les coordonnées x = 0,07w, y = -0,794w, z = 0,5w et le rayon R1 = 0,54w +0,08w et le rayon R2 = 0,54w -0,08w.

8. Prothèse de genou selon la revendication 7, **caractérisée en ce que** le rayon R1 est R1 = 0,54w + 0.03w et le rayon R2 est R2 = 0,54w - 0,03w.

9. Prothèse de genou selon la revendication 1 à 8 **caractérisée en ce que**, à chaque angle γ de flexion, une tangente T1 à la ligne de trace Lt1 au point de contact Pt1 est aussi la tangente de la ligne de trace Lt2 sur la surface sphérique « Sc2 » du fémur et en ce, à chaque angle de flexion l'emplacement de l'axe (12) de rotation instantanée est dans un plan E1, qui passe par le centre Mb de la sphère 1 médiale et est perpendiculaire à la tangente T1 de la ligne de trace Lt1 tridimensionnelle aux points de contact Pt1 du tibia.

10. Prothèse de genou selon l'une quelconque des revendications 1 à 9, construite comme prothèse totale de genou avec un composant fémoral, qui présente une rainure (9) similaire à une rainure patellaire anatomique, et avec un composant (2) du tibia ayant une forme appropriée de découpe (10) postérieure destinée à maintenir un des ligaments croisés ou les deux.

11. Prothèse de genou selon l'une quelconque des revendications 1 à 9, construite comme prothèse unicompartimentale latérale conçue pour maintenir une patelle naturelle et une rainure de patelle en combinaison avec l'articulation congruente entre le condyle medio-fémoral naturel et la géométrie convexe de la géométrie tibiale et méniscale.

12. Prothèse de genou selon la revendication 1 à 11, **caractérisée en ce que** les surfaces « Bi » et « Be » de guidage des composants tibial et fémoral sont moins congruentes pour la plage intermédiaire de l'angle γ de flexion, que pour les positions terminales d'extension totale et de flexion totale.

13. Prothèse de genou selon la revendication 1 à 12, **caractérisée en ce que** des surfaces 17, 18 coniques sont ajoutées sur le côté intérieur des lignes de trace Lt1 et Lt2 pour un support supplémentaire, qui ont leur centre au niveau du centre Mb de la sphère 1 médiale et qui ont les lignes de trace Lt1 et Lt2 comme générateur des cônes.
